# EUROPEAN PATENT SPECIFICATION

(11) **EP 0 670 162 B1**
(45) Date of publication and mention of the grant of the patent: **16.01.2002**
(21) Application number: 95301291.1
(22) Date of filing: 28.02.1995
(51) Int. Cl.: A61K 31/445, A61K 47/00, A61K 47/08

(54) **Pharmaceutical formulations containing raloxifere, a surfactant and a watersoluble diluent**
Pharmazeutische Zubereitungen enthaltend Raloxifen, einen oberflächenaktiven Stoff und ein wasserlösliches Verdünnungsmittel
Formulation pharmaceutique contenant le raloxifère, un agent surfactif et un diluant hydrosoluble

(30) Priority: 02.03.1994 US 204915
(43) Date of publication of application: 06.09.1995
(73) Proprietor: ELI LILLY AND COMPANY, Indianapolis, Indiana 46285 (US)
(72) Inventor: Gibson, Lowell Lee, Greenwood, Indiana 46142 (US); Hartauer, Kerry John, Indianapolis, Indiana 46278 (US); Stowers, Julian Larry, Indianapolis, Indiana 46256 (US); Sweetana, Stephanie Ann, Bloomington, Indiana 47401 (US); Thakkar, Arvind Lavji, Indianapolis Indiana 46260 (US)
(74) Representative: Tapping, Kenneth George

(56) References cited:
- CA-A- 2 101 356
- US-A- 4 418 068
- PHARMAC. THER., vol. 44,no. 3, 1989 pages 407-443, EWOUD J. VAN HOOGDALEM ET AL. 'Intestinal drug absorption enhancement: an overview'

## Description

Certain benzothiophenes of the formula 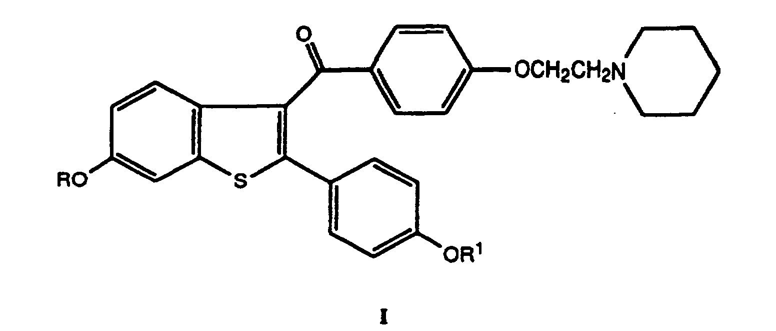 wherein
R and R¹ are independently hydrogen, COR², or R³;
R² is hydrogen, C₁-C₁₄ alkyl, C₁-C₃ chloroalkyl, C₁-C₃ fluoroalkyl, C₅-C₇ cycloalkyl, C₁-C₄ alkoxy, phenyl, or phenyl mono- or disubstituted with C₁-C₄ alkyl, C₁-C₄ alkoxy, hydroxy, nitro, chloro, fluoro, or tri(chloro or fluoro)methyl;
R³ is C₁-C₄ alkyl, C₅-C₇ cycloalkyl, or benzyl; or
a pharmaceutically-acceptable salt thereof; are nonsteriodal antiestrogens and antiandrogens. These compounds are useful in the treatment of mammary and prostatic tumors, and in the treatment of mammary and prostatic fibrocystic disease. The formula I compounds are described in U.S. Patent No. 4,418,068 (issued November 29, 1983). This patent described the preparation of these compounds, as well as their use for antiestrogen and antiandrogen therapy. The preparation of pharmaceutical compositions for antiestrogenic and antiandrogenic therapy was also described.

Raloxifene, which is 6-hydroxy-2-(4-hydroxyphenyl)-3-[4-(2-piperidinoethoxy)benzoyl]benzo[b]thiophene, is a particulary useful compound from this series of benzothiophenes. Raloxifene competitively inhibits estrogen action in a number of in vitro and *in vivo* models. Black, Jones, and Falcone, *Life Sci*., **32,** 1031-1036 (1983); Knecht, Tsai-Morris, and Catt, *Endocrinology,* **116**, 1771-1777 (1985); and Simard and Labrie, *Mol. Cell*. *Endocrinology,* **39,** 141-144 (1985). This compound also displays some estrogen-like actions in addition to its estrogen-antagonistic effects. Ortmann, Emons, Knuppen, and Catt, *Endocrinology,* **123,** 962-968 (1988). A recent report suggests that raloxifene is useful in the treatment of osteoporosis in postmenopausal women. Turner, Sato, and Bryant, *Journal of Clinical Investigation* (In Press).

Raloxifene compositions are dislosed in CA-A-2101356, while polyoxyethylene absorption accelerators are disclosed in Phamac. Ther., 44 (3), 1989, pp 407-443.

The formula I compounds may be administered as pharmaceutically-acceptable salts. A particularly useful pharmaceutically-acceptable salt of raloxifene is the hydrochloride salt. This salt form is easily prepared by the addition of hydrogen chloride to a solution of raloxifene in an organic solvent, such as tetrahydrofuran or methanol. Aqueous solubility of raloxifene hydrochloride, however, is far below what would be expected for an organic hydrochloride salt containing two phenolic hydroxyl groups. This poor solubility has somewhat limited the bioavailability of this preferred salt form. Another significant barrier to optimum and consistent absorption of raloxifene hydrochloride is its hydrophobicity.

To overcome the limited bioavailability, the present invention provides orally administerable pharmaceutical formulations comprising raloxifene, its esters or ethers, or a pharmaceutically-acceptable salt thereof, in combination with a hydrophilic carrier composition, such formulations having increased solubility in aqueous media. More particularly, the present invention provides an orally administerable pharmaceutical formulation comprising raloxifene, its esters or ethers, or a pharmaceutically-acceptable salt thereof, in combination with a surfactant, a water-soluble diluent, and optionally a hydrophilic binder. The present invention also provides pharmaceutical formulations further comprising a lubricant and a disintegrant.

The present invention provides orally administerable pharmaceutical formulations comprising raloxifene hydrochloride, in combination with a surfactant, a water-soluble diluent, and optionally a hydrophilic binder. Raloxifene is represented by the following formula: 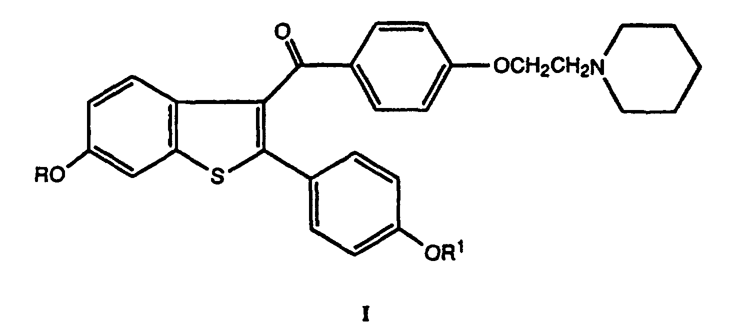 wherein R and R¹ are each hydrogen. Raloxifene is the compound wherein R and R¹ are hydrogen. The preparation of this compound is described in U.S. Patent No. 4,418,068.

The term "hydrophilic binder" represents binders commonly used in the formulation of pharmaceuticals, such as polyvinylpyrrolidone, polyethylene glycol, sucrose, dextrose, corn syrup, polysaccharides (including acacia, tragacanth, guar, and alginates), gelatin, and cellulose derivatives (including hydroxypropyl methylcellulose, hydroxypropyl cellulose, and sodium carboxymethylcellulose).

The term "surfactant", as used herein, represents ionic and nonionic surfactants or wetting agents commonly used in the formulation of pharmaceuticals, which are selected from the group consisting of ethoxylated castor oil, polyglycolyzed glycerides, acetylated monoglycerides, sorobitan fatty acid esters, poloxamers, polyoxyethylene sorbitan fatty acid esters, polyoxyethylene derivatives, monoglycerides or ethoxylated derivatives thereof, diglycerides or polyoxyethylene derivatives thereof and sodium docusate.

The term 'water-soluble diluent' represents compounds typically used in the formulation of pharmaceuticals, wherein the water-soluble diluent is selected from the group consisting of sugars (including lactose, sucrose, and dextrose) and polyols (including mannitol, xylitol, and sorbitol).

The term "disintegrant" represents compounds such as starches, clays, celluloses, alginates, gums, cross-linked polymers (such as cross-linked polyvinylpyrrolidone and cross-linked sodium carboxymethylcellulose), sodium starch glycolate, low-substituted hydroxypropyl cellulose, and soy polysaccharides. Preferably the disintegrant is a cross-linked polymer, more preferably cross-linked polyvinylpyrrolidone.

The term "lubricant" represents compounds frequently used as lubricants or glidants in the preparation of pharmaceuticals, such as talc, magnesium stearate, calcium stearate, stearic acid, colloidal silicon dioxide, magnesium carbonate, magnesium oxide, calcium silicate, microcrystalline cellulose, starches, mineral oil, waxes, glyceryl behenate, polyethylene glycol, sodium benzoate, sodium acetate, sodium chloride, sodium laurylsulfate, sodium stearyl fumarate, and hydrogenated vegetable oils. Preferably the lubricant is magnesium stearate or stearic acid, more preferably magnesium stearate.

While all of the formulations of the present invention have increased solubility in aqueous media and, therefore, greater bioavailability would be expected, certain formulations are preferred. Preferably, the surfactant is an anionic or nonionic surfactant. Representative surfactants from this preferred group include sodium docusate, ethoxylated castor oil, polyglycolyzed glycerides, acetylated monoglycerides, sorbitan fatty acid esters, poloxamers, polyoxyethylene sorbitan fatty acid esters, polyoxyethylene derivatives, monoglycerides or ethoxylated derivatives thereof, and diglycerides or polyoxyethylene derivatives thereof. Preferably, the water-soluble diluent is a sugar or polyol. When a hydrophilic binder is present, preferably the binder is sucrose, dextrose, corn syrup, gelatin, a cellulose derivative, or polyvinylpyrrolidone.

Certain formulations of the present invention are more preferred. More preferably, the surfactant is a nonionic surfactant, such as ethoxylated castor oil, polyglycolyzed glycerides, acetylated monoglycerides, sorbitan fatty acid esters, poloxamers, polyoxyethylene sorbitan fatty acid esters, polyoxyethylene derivatives, monoglycerides or ethoxylated derivatives thereof, and diglycerides or polyoxyethylene derivatives thereof. More preferably, the water-soluble diluent is a sugar, such as lactose, sucrose, and dextrose. More preferably, the hydrophilic binder is a cellulose derivative or polyvinylpyrrolidone.

Certain formulations of the present invention are most preferred. Most preferably, the surfactant is a polyoxyethylene sorbitan fatty acid ester, such as polysorbate 80. Most preferably, the water-soluble diluent is lactose. Most preferably the hydrophilic binder, when present, is polyvinylpyrrolidone.

The orally administerable compositions of the present invention are prepared and administered according to methods well known in pharmaceutical chemistry. *See* **Remington's Pharmaceutical Sciences,** 17th ed. (A. Osol ed., 1985). For example, the compositions of the present invention may be adminstered by means of solid dosage forms such as tablets and capsules. Preferably, the compositions are formulated as tablets. These tablets are prepared by wet granulation, by dry granulation, or by direct compression.

Tablets for this invention are prepared utilizing conventional tabletting techniques. A general method of manufacture involves blending raloxifene, its ester, ether, or a salt thereof, the water-soluble diluent, and optionally a portion of a disintegrant. This blend is then granulated with a solution of the hydrophilic binder and surfactant in water and/or organic solvent, such as methanol, ethanol, isopropanol, methylene chloride, and acetone, and milled if necessary. The granules are dried and reduced to a suitable size. Any other ingredients, such as lubricants, (e.g. magnesium stearate) and additional disintegrant, are added to the granules and mixed. This mixture is then compressed into a suitable size and shape using conventional tabletting machines such as a rotary tablet press. The tablets may be film coated by techniques well known in the art.

Capsules for this invention are prepared utilizing conventional encapsulating methods. A general method of manufacture involves blending raloxifene, its ester, ether, or salt thereof, the water-soluble diluent, and optionally a portion of a disintegrant. This blend is then granulated with a solution of the hydrophilic binder and surfactant in water and/or organic solvent, and milled if necessary. The granules are dried and reduced to a suitable size. Any other ingredients, such as a lubricant (e.g. colloidal silicon dioxide) are added to the granules and mixed. The resulting mixture is then filled into a suitable size hard-shell gelatin capsule using conventional capsule-filling machines.

The following formulation examples are illustrative only and are not intended to limit the scope of the invention in any way. Tablets may be prepared using the ingredients and procedures as described below:

### Formulation 1

| Ingredient | Weight (mg/tablet) |
|---|---|
| Raloxifene HCl | 200.00 |
| Polyvinylpyrrolidone | 15.75 |
| Polysorbate 80 | 5.25 |
| Lactose Anhydrous | 264.62 |
| Cross-linked polyvinylpyrrolidone | 31.50 |
| Stearic Acid | 5.25 |
| Magnesium Stearate | 2.63 |

The mixture of raloxifene HCl, lactose, and a portion of the cross-linked polyvinylpyrrolidone is granulated with an aqueous solution of the polyvinylpyrrolidone and polysorbate 80. The granules are dried, reduced to a suitable size, and mixed with stearic acid, magnesium stearate, and remaining cross-linked polyvinylpyrrolidone. The mixture is compressed into individual tablets yielding a tablet weight of 525 mg.

### Formulation 2

| Ingredient | Weight (mg/tablet) |
|---|---|
| Raloxifene HCl | 200.00 |
| Polyvinylpyrrolidone | 15.75 |
| Polysorbate 80 | 5.75 |
| Lactose Anhydrous | 132.06 |
| Dextrose | 132.06 |
| Cross-linked polyvinylpyrrolidone | 31.50 |
| Stearic acid | 5.25 |
| Magnesium Stearate | 2.63 |

The mixture of raloxifene HCl, lactose anhydrous, dextrose, and a portion of the cross-linked polyvinylpyrrolidone is granulated with an alcoholic solution of polyvinylpyrrolidone and polysorbate 80. The granules are dried, reduced to a suitable size, and mixed with magnesium stearate, stearic acid, and remaining cross-linked polyvinylpyrrolidone. The mixture is compressed into individual tablets yielding a tablet weight of 525 mg.

### Formulation 4

| Ingredient | Weight (mg/tablet) |
|---|---|
| Raloxifene HCl | 30.00 |
| Lactose Anhydrous | 144.00 |
| Lactose, Hydrous spray Dried | 36.00 |
| Polyvinylpyrrolidone | 12.00 |
| Polysorbate 80 | 2.40 |
| Cross-linked polyvinylpyrrolidone | 14.40 |
| Magnesium Stearate | 1.20 |

The mixture of raloxifene HCl, lactose anhydrous, spray-dried hydrous lactose, and a portion of the cross-linked polyvinylpyrrolidone is granulated with an aqueous solution of polyvinylpyrrolidone and polysorbate 80. The granules are dried, reduced to a suitable size, and mixed with magnesium stearate and remaining cross-linked polyvinylpyrrolidone. The mixture is compressed into individual tablets yielding a tablet weight of 240 mg.

### Formulation 5

| Ingredient | Weight (mg/tablet) |
|---|---|
| Raloxifene HCl | 30.00 |
| Lactose Anhydrous | 160.00 |
| Hydroxypropyl Cellulose | 11.00 |
| Poloxamer | 7.00 |
| Cross-linked sodium carboxymethylcellulose | 23.00 |
| Stearic Acid | 7.00 |
| Magnesium Stearate | 2.00 |

The mixture of raloxifene HCl, anhydrous lactose, and cross-linked sodium carboxymethylcellulose is granulated with an aqueous solution of poloxamer and hydroxypropyl cellulose. The granules are dried, reduced to a suitable size, and mixed with stearic acid and magnesium stearate. The mixture is then compressed into individual tablets yielding a tablet weight of 240 mg.

### Formulation 7

| Ingredient | Weight (mg/tablet) |
|---|---|
| Raloxifene HCl | 60.00 |
| Lactose Anhydrous | 156.00 |
| Polyvinylpyrrolidone | 7.20 |
| Polysorbate 80 | 7.20 |
| Cross-linked polyvinylpyrrolidone | 7.20 |
| Magnesium Stearate | 2.40 |

The mixture of raloxifene HCl, lactose anhydrous, and cross-linked polyvinylpyrrolidone is granulated with an aqueous solution of polyvinylpyrrolidone and polysorbate 80. The granules are dried, reduced to a suitable size, and mixed with magnesium stearate. The mixture is then compressed into individual tablets yielding a tablet weight of 240 mg.

### Formulation 8

| Ingredient | Weight (mg/tablet) |
|---|---|
| Raloxifene HCl | 60.00 |
| Lactose Anhydrous | 120.00 |
| Lactose, hydrous spray-dried | 30.00 |
| Polyvinylpyrrolidone | 12.00 |
| Polysorbate 80 | 2.40 |
| Cross-linked polyvinylpyrrolidone | 14.40 |
| Magnesium Stearate | 1.20 |

The mixture of raloxifene HCl, lactose anhydrous, spray-dried hydrous lactose, and a portion of the cross-linked polyvinylpyrrolidone is granulated with an aqueous' solution of polyvinylpyrrolidone and polysorbate 80. The granules are dried, reduced to a suitable size, and mixed with magnesium stearate and remaining cross-linked polyvinylpyrrolidone. The mixture is then compressed into individual tablets yielding a tablet weight of 240 mg.

### Formulation 9

| Ingredient | Weight (mg/tablet) |
|---|---|
| Raloxifene HCl | 60.00 |
| Mannitol | 77.00 |
| Dextrose | 73.00 |
| Hydroxypropyl methylcellulose | 7.00 |
| Polysorbate 80 | 4.00 |
| Sodium Starch Glycolate | 14.00 |
| Stearic Acid | 4.00 |
| Magnesium Stearate | 1.00 |

The mixture of raloxifene HCl, mannitol, dextrose, and sodium starch glycolate is granulated with an aqueous solution of polysorbate 80 and hydroxypropyl methylcellulose. The granules are dried, reduced to a suitable size, and mixed with stearic acid and magnesium stearate. The mixture is then compressed into individual tablets yielding a tablet weight of 240 mg.

### Formulation 10

| Ingredient | Weight (mg/tablet) |
|---|---|
| Raloxifene HCl | 150.00 |
| Lactose,anhydrous | 41.00 |
| Lactose, hydrous spray dried | 10.25 |
| Polyvinylpyrrolidone | 11.50 |
| Polysorbate 80 | 2.30 |
| Cross-linked polyvinylpyrrolidone | 13.80 |
| Magnesium Stearate | 1.15 |

The mixture of raloxifene HCl, anhydrous lactose, hydrous spray-dried lactose, and a portion of the cross-linked polyvinylpyrrolidone is granulated with an aqueous solution of polyvinylpyrrolidone and polysorbate 80. The granules are dried, reduced to a suitable size, and mixed with magnesium stearate and the remaining cross-linked polyvinylpyrrolidone. The mixture is then compressed into individual tablets yielding a tablet weight of 230 mg.

### Formulation 11

| Ingredient | Weight (mg/tablet) |
|---|---|
| Raloxifene HCl | 150.00 |
| Lactose, hydrous spray-dried | 56.00 |
| Polyvinylpyrrolidone | 7.00 |
| Polysorbate 80 | 1.20 |
| Cross-linked polyvinylpyrrolidone | 13.80 |
| Magnesium Stearate | 2.00 |

The mixture of raloxifene HCl, hydrous spray-dried lactose, and a portion of the cross-linked polyvinylpyrrolidone is granulated with an aqueous solution of polyvinylpyrrolidone and polysorbate 80. The granules are dried, reduced to a suitable size and mixed with magnesium stearate and remaining cross-linked polyvinylpyrrolidone. The mixture is then compressed into individual tablets yielding a tablet weight of 230 mg.

### Formulation 12

| Ingredient | Weight (mg/tablet) |
|---|---|
| Raloxifene HCl | 150.00 |
| Lactose, anhydrous | 52.40 |
| Polysorbate 80 | 4.60 |
| Polyvinylpyrrolidone | 11.50 |
| Polyethylene Glycol 8000 | 11.50 |

The mixture of raloxifene HCl and anhydrous lactose is granulated with an aqueous solution of polysorbate 80 and polyvinylpyrrolidone. The granules are dried, reduced to a suitable size, and mixed with the polyethylene glycol 8000. The mixture is then compressed into individual tablets yielding a tablet weight of 230 mg.

Capsules may be prepared using the ingredients and procedures as described below:

### Formulation 13

| Ingredient | Weight (mg/capsule) |
|---|---|
| Raloxifene HCl | 30.00 |
| Lactose, hydrous spray-dried | 178.30 |
| Sodium laurylsulfate | 4.60 |
| Cross-linked polyvinylpyrrolidone | 9.20 |
| Hydroxypropyl methylcellulose | 6.90 |
| Colloidal Silicon Dioxide | 1.00 |

The mixture of raloxifene HCl, hydrous spray-dried lactose, and cross-linked polyvinylpyrrolidone is granulated with an aqueous solution of sodium laurylsulfate and hydroxypropyl methylcellulose. The granules are dried, reduced to a suitable size, and mixed with colloidal silicon dioxide. This mixture is then filled into Size 3 hard-shell gelatin capsules utilizing conventional encapsulating equipment, with each capsule containing 230 mg of the final mixture.

## Claims

1. An orally administerable pharmaceutical formulation consisting essentially of raloxifene hydrochloride, in combination with a surfactant, polyvinylpyrrolidone and a water-soluble diluent, wherein:
the surfactant is selected from the group consisting of sodium docusate, ethoxylated castor oil, polyglycolysed glycerides, acetylated monoglycerides, sorbitan fatty acid esters, poloxamers, polyoxyethylene sorbitan fatty acid esters, polyoxyethylene derivatives, monoglycerides or ethoxylated derivatives thereof, and diglycerides or polyoxyethylene derivatives thereof; and
the water-soluble diluent is a polyol or sugar.

2. The formulation of claim 1, wherein the surfactant is a polyoxyethylene sorbitan fatty acid ester.

3. The formulation of claim 1 or claim 2, wherein the surfactant is polysorbate 80.

4. The formulation of any one of claims 1 to 3, wherein the water-soluble diluent is a sugar.

5. The formulation of any one of claims 1 to 4, wherein the water-soluble diluent is lactose.

6. The formulation of any one of claims 1 to 5, further comprising a lubricant and a disintegrant.

7. The formulation of claim 6, wherein the lubricant is magnesium stearate or stearic acid, and the disintegrant is cross-linked polyvinylpyrrolidone.

8. An orally administerable pharmaceutical formulation consisting essentially of raloxifene hydrochloride in combination with polysorbate 80, cross-linked polyvinylpyrrolidone, lactose, polyvinylpyrrolidone and magnesium stearate.

9. The formulation of any one of claims 1 to 10 further comprising a film coating.

## Patentansprüche

1. Oral verabreichbare pharmazeutische Formulierung, die im wesentlichen aus Raloxifenhydrochlorid in Kombination mit einem oberflächenaktiven Mittel, Polyvinylpyrrolidon und einem wasserlöslichen Verdünnungsmittel besteht, worin das oberflächenaktive Mittel aus der Gruppe ausgewählt ist, die besteht aus Natriumdocusat, ethoxyliertem Rizinusöl, polyglycolysierten Glyceriden, acetylierten Monoglyceriden, Sorbitanfettsäureestern, Poloxameren, Polyoxyethylensorbitanfettsäureestern, Polyoxyethylenderivaten, Monoglyceriden oder ethoxylierten Derivaten hiervon und Diglyceriden oder Polyoxyethylenderivaten hiervon und das wasserlösliche Verdünnungsmittel ein Polyol oder Zucker ist.

2. Formulierung nach Anspruch 1, worin das oberflächenaktive Mittel ein Polyoxyethylensorbitanfettsäureester ist.

3. Formulierung nach Anspruch 1 oder 2, worin das oberflächenaktive Mittel Polysorbat 80 ist.

4. Formulierung nach einem der Ansprüche 1 bis 3, worin das wasserlösliche Verdünnungsmittel ein Zucker ist.

5. Formulierung nach einem der Ansprüche 1 bis 4, worin das wasserlösliche Verdünnungsmittel Lactose ist.

6. Formulierung nach einem der Ansprüche 1 bis 5, die ferner ein Gleitmittel und ein Zerfallhilfsmittel enthält.

7. Formulierung nach Anspruch 6, worin das Gleitmittel Magnesiumstearat oder Stearinsäure ist und das Zerfallhilfsmittel quervernetztes Polyvinylpyrrolidon ist.

8. Oral verabreichbare pharmazeutische Formulierung, die im wesentlichen aus Raloxifenhydrochlorid in Kombination mit Polysorbat 80, quervernetztem Polyvinylpyrrolidon, Lactose, Polyvinylpyrrolidon und Magesiumstearat besteht.

9. Formulierung nach einem der Ansprüche 1 bis 10, die ferner eine Filmbeschichtung umfaßt.

## Revendications

1. Formulation pharmaceutique pouvant être administrée par voie orale, constituée essentiellement de chlorhydrate de raloxifène, en combinaison avec un agent tensioactif, de la polyvinylpyrrolidone et un diluant soluble dans l'eau, dans laquelle :
l'agent tensioactif est choisi parmi le groupe constitué de docusate de sodium, d'huile de ricin éthoxylée, de glycérides polyglycolysés, de monoglycérides acétylés, d'esters d'acide gras de sorbitane, de poloxamères, d'esters d'acide gras de polyoxyéthylènesorbitane, de dérivés de polyoxyéthylène, de monoglycérides ou de dérivés éthoxylés de ceux-ci, de diglycérides ou de dérivés polyoxyéthylène de ceux-ci ; et
le diluant soluble dans l'eau est un polyol ou un sucre.

2. Formulation de la revendication 1, dans laquelle l'agent tensioactif est un ester d'acide gras de polyoxyéthylènesorbitane.

3. Formulation de la revendication 1 ou de la revendication 2, dans laquelle l'agent tensioactif est du polysorbate 80.

4. Formulation de l'une quelconque des revendications 1 à 3, dans laquelle le diluant soluble dans l'eau est un sucre.

5. Formulation de l'une quelconque des revendications 1 à 4, dans laquelle le diluant soluble dans l'eau est du lactose.

6. Formulation de l'une quelconque des revendications 1 à 5, comprenant en outre un lubrifiant et un agent de désintégration.

7. Formulation de la revendication 6, dans laquelle le lubrifiant est du stéarate de magnésium ou de l'acide stéarique et l'agent de désintégration est de la polyvinylpyrrolidone réticulée.

8. Formulation pharmaceutique pouvant être administrée par voie orale constituée essentiellement de chlorhydrate de raloxifène en combinaison avec du polysorbate 80, de la polyvinylpyrrolidone réticulée, du lactose, de la polyvinylpyrrolidone et du stéarate de magnésium.

9. Formulation de l'une quelconque des revendications 1 à 8, comprenant en outre un revêtement de film.
